# EUROPEAN PATENT APPLICATION

(11) **EP 3 822 336 A1**
(43) Date of publication of application: **19.05.2021**
(21) Application number: 19834416.0
(22) Date of filing: 09.07.2019
(51) Int. Cl.: C12M 1/00, C12N 5/07

(54) **CELL CULTURE SHEET**

(30) Priority: 10.07.2018 JP 2018131132
(71) Applicant: Nippon Shokubai Co., Ltd., Osaka-shi, Osaka 541-0043 (JP)
(72) Inventor: MAKINO, Tomomi, Suita-shi, Osaka 564-0034 (JP); SHIMA, Fumiaki, Suita-shi, Osaka 564-0034 (JP); NAKAZAWA, Koji, Kitakyushu-shi, Fukuoka 808-0135 (JP)
(74) Representative: Lederer & Keller Patentanwälte Partnerschaft mbB
(86) International application number: PCT/JP2019/027228
(87) International publication number: WO 2020/013207

(57) **Abstract**

The present invention provides a cell culture sheet comprising a plurality of recesses each having an opening of 1000 µm or less in diameter, wherein each recess has an inner circumferential face and a bottom face, wherein the inner circumferential face has a non-cell adhesive surface, and wherein the bottom face has a cell-adhesive surface; and a method for producing a cell culture sheet, the method comprising laminating of a layer having a non-cell adhesive surface and a layer having a cell-adhesive surface, wherein the layer having a non-cell adhesive surface has a plurality of through-holes of 1000 µm or less in diameter. The cell culture sheet of the present invention can be prepared simply and assists efficient cell culture operation, and therefore can preferably be used in the field of cell-based pharmaceutical preparations, for example, spheroid-containing pharmaceutical preparations etc.

## Description

### TECHNICAL FIELD

The present invention relates to a cell culture sheet. More particularly, the present invention relates to a cell culture sheet, a method for producing the cell sheet, a cell culture tool comprising the sheet, and a method for producing a spheroid using the sheet or the tool.

### BACKGROUND ART

In the field of cell culture, three-dimensional cell culture technology has recently drawn attention.

For example, Patent Literature 1 discloses a microchip having a plurality of cell-holding cavities, each of which has a bottom face comprising an adhesive region having cell adhesiveness and a non-adhesive region surrounding the adhesive region and having non-cell adhesiveness. Patent Literature 1 states that the use of such a microchip enables the formation of cellular tissue structures of a homogeneous shape and size. Patent Literature 2 discloses a method for culturing cells on a porous film having through-holes, the porous film having a surface having a cell-adhesive region and a non-cell adhesive region. Patent Literature 2 states that this method enables efficient culture of three-dimensional tissue structures in concurrence with smooth supply of fresh culture medium and smooth elimination of wastes.

### CITATION LIST

### Patent Literature

Patent Literature 1: JP-A 2006-121991
Patent Literature 2: JP-A 2008-199962

### SUMMARY OF INVENTION

### TECHNICAL PROBLEM

However, conventional culture methods disadvantageously require cumbersome preparation of cell culture tools and are unsatisfactory in terms of the homogeneity of the resulting cellular tissue structures. For these reasons, novel technologies have been desired.

An object of the present invention is to provide a novel cell culture sheet.

The present invention is intended to provide a cell culture sheet that can be prepared simply and assists efficient cell culture operation; a method for producing the cell culture sheet; a cell culture tool comprising the sheet; and a method for producing spheroids using the sheet or the tool.

### SOLUTION TO PROBLEM

The present inventors conducted extensive research to achieve the above-mentioned object. As a result, the present inventors designed a cell culture sheet having a specific configuration and found that such a cell culture sheet can be prepared simply, facilitates cell culture operation such as medium change and cell seeding, and enables more homogeneous production of cell aggregates (spheroids). Based on this finding, the present inventors completed the present invention.

That is, the present invention relates to the following (1) to (10).
[1] A cell culture sheet comprising a plurality of recesses each having an opening of 1000 µm or less in diameter,
   wherein each recess has an inner circumferential face and a bottom face,
   wherein the inner circumferential face has a non-cell adhesive surface, and
   wherein the bottom face has a cell-adhesive surface.
[2] The cell culture sheet according to the above [1], wherein the cell culture sheet is a laminate comprising a layer having the non-cell adhesive surface and a layer having the cell-adhesive surface, wherein the layer having the non-cell adhesive surface has through-holes.
[3] The cell culture sheet according to the above [2], further comprising an adhesive layer between the layer having the non-cell adhesive surface and the layer having the cell-adhesive surface.
[4] The cell culture sheet according to any one of the above [1] to [3], wherein the cell-adhesive surface comprises a synthetic resin.
[5] The cell culture sheet according to any one of the above [1] to [4], wherein the cell-adhesive surface comprises a resin composition containing a polyimide.
[6] The cell culture sheet according to any one of the above [1] to [5], wherein the recesses are formed in a shape tapered to the bottom face.
[7] The cell culture sheet according to any one of the above [1] to [6], wherein the number of the recesses formed per unit area (cm²) is 10 to 1000.
[8] A method for producing a cell culture sheet, the method comprising laminating of a layer having a non-cell adhesive surface and a layer having a cell-adhesive surface, wherein the layer having a non-cell adhesive surface has a plurality of through-holes of 1000 µm or less in diameter.
[9] A cell culture tool, comprising the cell culture sheet according to any one of the above [1] to [7].
[10] A method for culturing spheroids, the method comprising culturing spheroids using the cell culture sheet or cell culture tool according to any one of the above [1] to [7] and [9].

### ADVANTAGEOUS EFFECTS OF INVENTION

The cell culture sheet of the present invention can be prepared simply and assists efficient cell culture operation.

Further advantageously, the cell culture sheet of the present invention enables more homogeneous production of cell aggregates (spheroids) and even facilitates quality control of cells.

### BRIEF DESCRIPTION OF DRAWINGS

Fig. 1 is a schematic view of a cross-sectional structure of one embodiment of the cell culture sheet of the present invention.
Fig. 2 is a schematic view of a cross-sectional structure of one embodiment of the cell culture sheet of the present invention.
Fig. 3 is a schematic view of a cross-sectional structure of one embodiment of the cell culture sheet of the present invention.
Fig. 4 is a schematic view of one embodiment of the cell culture sheet of the present invention on which a cell is cultured.
Fig. 5 is a schematic view of one embodiment of the cell culture sheet of the present invention on which a cell is cultured.
Fig. 6 is a schematic view of a cross-sectional structure of one embodiment of the cell culture sheet of the present invention.
Fig. 7 shows images of spheroids obtained using the cell culture sheet of the present invention.
Fig. 8 is a graph showing the fluid diameter of spheroids obtained using the cell culture sheet of the present invention.
Fig. 9 is a graph showing the equivalent circle diameter of spheroids obtained using the cell culture sheet of the present invention.
Fig. 10 is a graph showing the circularity of spheroids obtained using the cell culture sheet of the present invention.
Fig. 11 is a layout of sections in the cell culture sheet used in Test Examples 2 and 3.
Fig. 12 is a graph showing the equivalent circle diameter of spheroids obtained in each section of the cell culture sheet of the present invention.
Fig. 13 is a graph showing the equivalent circle diameter of spheroids obtained in each section of the cell culture sheet of the present invention.
Fig. 14 shows images of spheroids obtained using the cell culture sheets of Examples 2 to 5.

### DESCRIPTION OF EMBODIMENTS

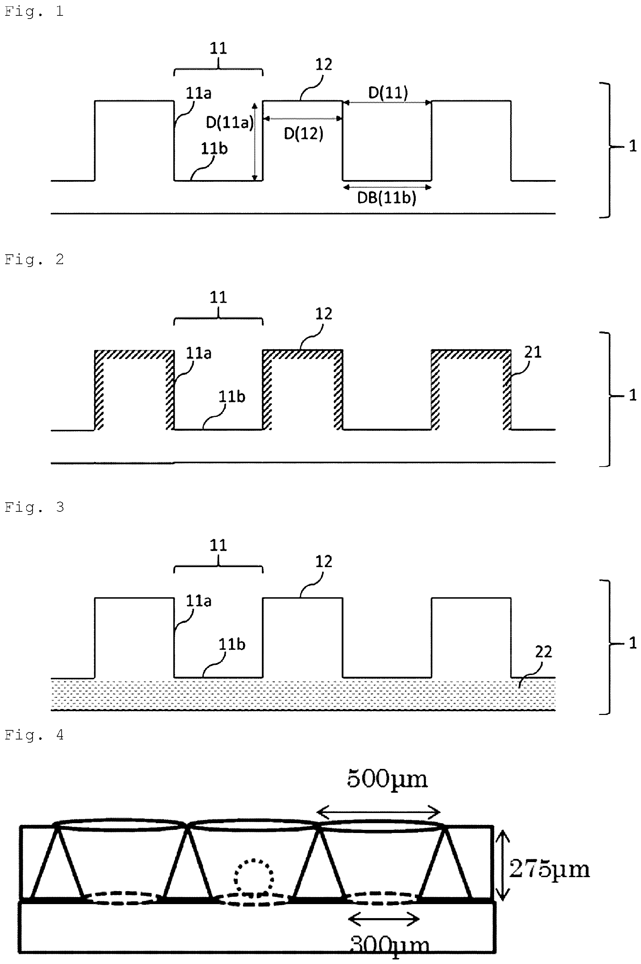

The cell culture sheet of the present invention comprises a plurality of recesses each having an opening of 1000 µm or less in diameter, wherein each recess has an inner circumferential face and a bottom face, wherein the inner circumferential face has a non-cell adhesive surface, and wherein the bottom face has a cell-adhesive surface. Here, the structure of the cell culture sheet of the present invention is described using a vertical cross-sectional view through recesses of the sheet. As shown in the schematic view of Fig. 1, a plurality of recesses 11 are present at a sheet surface 12, and each recess 11 has an inner circumferential face 11a and a bottom face 11b. The recess 11 is a space used for cellular tissue structure (spheroid) formation.

The cell culture sheet of the present invention has a plurality of recesses at the sheet surface. For example, in Fig. 1, a plurality of recesses 11 are formed at the sheet surface 12. The number of recesses varies with the area (dimension) of the sheet and the type of the cells to be cultured and cannot be definitely determined, but an appropriate number for each individual case can be selected according to the common technical knowledge. For example, the minimum number per unit area (cm²) may be 1, but is not limited thereto, and may be 10, 20, 30, 50, etc. The maximum number per unit area (cm²) may be 1000, 500, 300, 200, 100, etc. The total number of recesses at the sheet surface can be determined as appropriate and is, for example, 10 or more, 100 or more, 1000 or more, 10000 or more, 50000 or more, etc.

The shape of the opening of the recess is not limited to a circle and may be, for example, a polygon or an ellipse. The size of the opening is up to 1000 µm in diameter, and an appropriate size can be selected for the size of the cell to be cultured according to the common technical knowledge. In the present invention, the size of the opening is a diameter (maximum length) of a bounding circle enclosing the opening regardless of the shape of the opening. The size of the opening is, for example, the length represented by D (11) in Fig. 1. The size of the opening is, for example, within the range of 10 to 1000 µm, 10 to 700 µm, 10 to 600 µm, or 10 to 500 µm in diameter.

The shape of the bottom face of the recess is not limited to a circle and may be, for example, a polygon or an ellipse. In addition, the shape of the bottom face of the recess may be the same as or different from the shape of the opening. The size (length) of the bottom face may be the same as or different from the size of the opening. The size of the bottom face may be smaller than the size of the opening or greater than the size of the opening. The size of the bottom face is, for example, the length represented by DB(11b) in Fig. 1. The size of the bottom face is, for example, within the range of 10 to 1000 µm, 10 to 700 µm, 10 to 600 µm, 10 to 500 µm, 10 to 400 µm, or 10 to 300 µm in diameter. For example, in the case where the size of the bottom face is smaller than the size of the opening, the recess is formed in a shape tapered to the bottom face.

The ratio of the size of the bottom face to the size of the opening (the size of the bottom face/the size of the opening) is not particularly limited and is, for example, 5/1 to 1/5, 3/1 to 1/3, or 1/1 to 1/2 in view of ease of cell seeding and collection.

The distance (gap) between adjacent openings is, for example, the length represented by D(12) in Fig. 1. The distance is not particularly limited and can be determined as appropriate for the desired cell culture. For example, the distance is a finite value in the range of 800 µm or less, 700 µm or less, 600 µm or less, 500 µm or less, 300 µm or less, 200 µm or less, 100 µm or less, etc.

The depth of the recess can be determined as appropriate for the size of the cell to be cultured according to the common technical knowledge. The depth of the recess is, for example, the length represented by D(11a) in Fig. 1. For example, the depth of the recess is within the range of 10 to 300 µm or 10 to 1000 µm.

The ratio of the size of the opening to the depth of the recess (the size of the opening/the depth of the recess) is not particularly limited and is, for example, 5/1 to 1/5, 3/1 to 1/3, or 1/2 to 1/2 in view of ease of cell seeding and collection. When the ratio is within this range, cells are unlikely to accidentally jump out of the recess, and operation such as cell collection and degassing can easily be done.

The thickness of the bottom face of the recess is not particularly limited, and an appropriate thickness can be selected according to the common technical knowledge.

The inner circumferential face of the recess has a non-cell adhesive surface, and the bottom face of the recess has a cell-adhesive surface. This structure enables more homogeneous production of cellular tissue structures. For example, in Fig. 1, the inner circumferential face 11a has a non-cell adhesive surface, and the bottom face 11b has a cell-adhesive surface.

The non-cell adhesive surface is defined, for example, as the surface described as follows. When cells in a solution used for culture sediment on the surface, the cells almost completely maintain their shape and do not adhere to the surface at all or even if they temporarily adhere to the surface weakly, they spontaneously come off afterwards. The non-cell adhesive surface is, for example, formed of a non-cell adhesive substance immobilized physically or chemically on a substrate surface which constitutes the recesses. The substrate itself may be formed of a non-cell adhesive substance. For example, in the case where the non-cell adhesive substance is immobilized on the surface, as shown in the schematic view of Fig. 2, the non-cell adhesive substance 21 is immobilized at the sheet surface 12 and the inner circumferential face 11a of the recess 11.

The non-cell adhesive substance is not particularly limited as long as the cells used for culture do not adhere to the substance or as long as the substance does not bind to cell surface molecules, such as proteins and sugar chains, present on the cell membrane of the cells used for culture. The non-cell adhesive substance may or may not be biocompatible. The non-cell adhesive substance may be hydrophobic or hydrophilic. The non-cell adhesive substance may be, for example, ultra-water repellent (ultra-hydrophobic) or ultra-hydrophilic. For cell adhesion prevention, homogeneous spheroid production, efficient spheroid formation, etc., hydrophobic (particularly, ultra-hydrophobic) [e.g., more hydrophobic (particularly, ultra-hydrophobic) as compared with a hydrophobic or hydrophilic (particularly, hydrophobic) cell-adhesive surface or a resin which constitutes such a cell-adhesive surface (further the contact angle thereof)] substances are particularly preferred. In addition, hydrophilic (particularly, ultra-hydrophilic) [e.g., more hydrophilic (particularly, ultra-hydrophilic) as compared with a hydrophilic or hydrophobic (particularly, hydrophobic) cell-adhesive surface or a resin which constitutes such a cell-adhesive surface (further the contact angle thereof)] substances are also preferred.

Examples of such a substance include compounds, such as polyethylene glycol and its derivatives, 2-methacryloyloxyethyl phosphorylcholine (MPC), polyhydroxyethyl methacrylate (poly-HEMA), and segmented polyurethane (SPC); and proteins (albumin etc.) harvested from the living body. From these substances, an appropriate one can be selected according to the kind of the cells. In particular, 2-methacryloyloxyethyl phosphorylcholine (MPC) is preferred because this compound has favorable adhesion to a synthetic resin used for the substrate and contributes to simplifying the production process of the cell culture sheet and to more homogeneous production of cellular tissue structures.

The substance may be modified as appropriate for ease of handling, the desired level of hydrophobicity (e.g., ultra-hydrophobicity) or hydrophilicity (e.g., ultra-hydrophilicity), etc. For example, a hydrophilic substance may be subjected to crosslinking etc. so that it has both hydrophilicity and low water solubility. In another example, a raw material (e.g., hydrophobic or hydrophilic material) may be hydrophobized or hydrophilized (e.g., subjected to introduction of a hydrophobic or hydrophilic group etc.) to yield a material having the desired hydrophobicity or hydrophilicity.

For immobilization of the non-cell adhesive substance on the substrate surface, various method can be used. For example, a solution containing the non-cell adhesive substance may be applied and dried on the substrate surface; the non-cell adhesive substance may be melted and compression-bonded to the substrate surface; the non-cell adhesive substance may be applied on the substrate surface and cured with energy ray such as UV; the functional group of the non-cell adhesive substance may be covalently bonded to the functional group of the substrate by chemical reaction (e.g., condensation between certain functional groups, such as a carboxyl group and an amino group); or the thiol group of the non-cell adhesive substance may be bonded to a thin film of metal (platinum, gold, etc.) formed on the substrate in advance. The thickness of a layer of the non-cell adhesive substance immobilized on the substrate surface is not particularly limited and is, for example, 0.01 to 1000 µm.

The proportion of the non-cell adhesive surface of the inner circumferential face of the recess is not particularly limited and is preferably at a level that would result in less adhesion of cultured cells. For example, the proportion is preferably 90% or more, more preferably 95% or more, and particularly preferably 100% of the area of the inner circumferential face.

In order to estimate whether the non-cell adhesive surface is effective to enhance the homogeneity in size of cellular tissue structures and the circularity of cellular tissue structures, surface properties such as static water contact angle (described later) can be used as an indicator. For example, in the case where the non-cell adhesive surface is a hydrophobic surface formed of the hydrophobic substance described above, the static water contact angle is preferably 90° or more, more preferably 93° or more, and still more preferably 95° or more. The static water contact angle may be 150° or less and is preferably 130° or less, more preferably 120° or less.

On the other hand, in the case of a hydrophilic surface, the static water contact angle is preferably 65° or less, more preferably 55° or less, and still more preferably 50° or less. The static water contact angle may be 0° or more and is preferably 5° or more, more preferably 10° or more.

For example, when a highly hydrophobic MPC (or a surface formed of such an MPC) is used, the static water contact angle will be, for example, 90° or more or 100° or more.

The static water contact angle may be a static water contact angle measured using a non-cell adhesive surface or a static water contact angle measured using a non-cell adhesive substance (or a substance constituting a non-cell adhesive surface). The static water contact angle may be measured by, for example, the method described later etc.

The cell-adhesive surface is defined, for example, as the surface described as follows. When cells in a solution used for culture sediment on the surface, the cells adhere to the surface at certain points of attachment. In other words, cells are immobilized on the surface at an adhesion strength that allows easy detachment of the cells by pipetting or other liquid flows. In even other words, the cell-adhesive surface is, for example, a surface on which cells adhere at a certain strength to form a steric or three-dimensional tissue structure, such as a layered structure or a spheroid, rather than a surface on which cells are two-dimensionally maintained and proliferated in an adherent state. The non-cell adhesive surface is, for example, formed of a cell-adhesive substance disposed or immobilized physically or chemically on a substrate surface which constitutes the bottom faces of the recesses. The cell-adhesive substance may be disposed at regions other than the recesses. The substrate itself may be formed of the cell-adhesive substance. For example, in the case where the substrate itself is formed of a cell-adhesive substance, as shown in the schematic view of Fig. 3, the bottom face 11b of the recess 11 may be a part of a layer of the cell-adhesive substance 22.

The cell-adhesive substance is not particularly limited as long as the cells used for culture adhere to the substance or as long as the substance binds to cell surface molecules, such as proteins and sugar chains, present on the cell membrane of the cells used for culture. The cell-adhesive substance may be hydrophilic or hydrophobic. In view of cell adhesiveness, spheroid-forming efficiency, etc., hydrophilic (particularly, not ultra-hydrophilic) or hydrophobic (particularly, not ultra-hydrophobic) substances are preferred, and hydrophobic substances are more preferred. The cell adhesiveness of the cell-adhesive substance may be at a level that can prevent cells from accidentally jumping out of the recess. Examples of the cell-adhesive substance include substances harvested from the living body and synthetic substances, for example, proteins (collagen, fibronectin, laminin, etc.) and synthetic resins (fluorine resins, polyimide resins, polysulfone, polyether sulfone, polydimethylsiloxane, a mixture thereof, etc.). In the case where synthetic resins are used as the cell adhesive substance, an easy-to-handle cell culture sheet can be produced due to the strength and heat resistance of synthetic resins. Among synthetic resins, polyimide resins are preferred. The benefits of using polyimide resins are as follows: they are biocompatible; cellular tissue structures in an adherent state can be obtained; more homogeneous production of cellular tissue structures can be achieved; and medium change can easily be done because polyimide resins have moderate cell-adhesiveness for various types of cells. When nonbiological components such as polyimide resins are used in the cell culture sheet of the present invention, the cellular tissue structures (spheroids) obtained using the cell culture sheet of the present invention can readily be applied to the fields including regenerative medicine and drug development.

The polyimide resin can be, for example, a polyimide resin having a structural unit represented by the formula (I) shown below. For efficient spheroid formation, a resin having a fluorine atom in the molecule is preferred, and a fluorine-containing polyimide (fluorine-containing polyimide resin) is more preferred. The polyimide resin used in the present invention can typically be obtained by imidization of a polyamic acid obtained by polymerization of one or more kinds of acid dianhydrides and one or more kinds of diamines. The polyimide resin may contain a polyamic acid as a partial chemical structure. The polyimide resin may be produced by any known method. For example, a two-step synthesis method can be used. Briefly, a polyamic acid is first synthesized as a precursor, and the polyamic acid is then converted into a polyimide acid. The polyamic acid used as a precursor of the polyimide resin may be a polyamic acid derivative. Examples of the polyamic acid derivative include polyamic acid salts, polyamic acid alkyl esters, polyamic acid amides, polyamic acid derivatives from bismethylidene pyromellitide, polyamic acid silyl esters, polyamic acid isoimides, etc. Examples of the polyimide include polyimides derived from a combination of an acid anhydride, such as pyromellitic acid dianhydride, biphenyl tetracarboxylic dianhydride, or benzophenone tetracarboxylic dianhydride, and a diamine, such as oxydiamine, p-phenylenediamine, m-phenylenediamine, or benzophenonediamine. Examples of the resin containing a fluorine atom include fluorine-containing polyimide resins having a structural unit represented by the formula (I) shown below, such as 4,4'-(hexafluoroisopropylidene)diphthalic anhydride (6FDA)/1,4-bis(aminophenoxy)benzene (TPEQ) copolymer, 6FDA/4,4'-oxydiphthalic anhydride (ODPA)/TPEQ copolymer, 4,4'-(4,4'-isopropylidenediphenoxy)diphthalic acid (BPADA)/2,2-bis[4-(4-aminophenoxy)phenyl]hexafluoropropane (HFBAPP), 6FDA/2,2-bis(4-(4-aminophenoxy)phenyl)propane (BAPP) copolymer, 6FDA/2,2'-bis(trifluoromethyl)benzidine (TFMB) copolymer, 6FDA/4,4'-diaminodiphenyl ether (ODA) copolymer, 6FDA/4,4'-bis(4-aminophenoxy)biphenyl (BAPB) copolymer, etc.; ethylene-tetrafluoroethylene copolymers; etc.

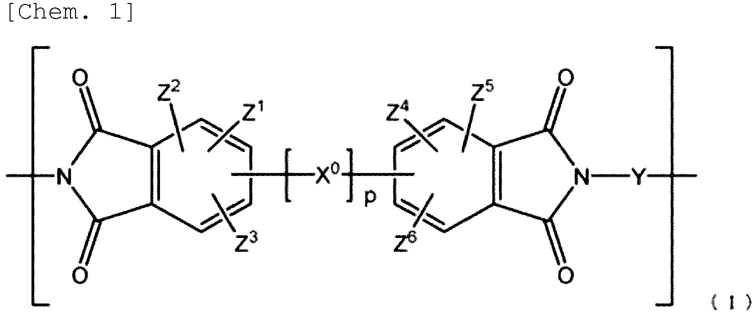

In the above formula (I), X⁰ represents an oxygen atom, a sulfur atom, or a divalent organic group;
Y represents a divalent organic group;
Z¹, Z², Z³ Z⁴, Z⁵, and Z⁶ independently represent a hydrogen atom, a fluorine atom, a chlorine atom, a bromine atom, or an iodine atom; and
p is 0 or 1.

In the polyimide resin, the chemical structure represented by formula (I) maybe different or identical for each structural unit. At least one of X⁰, Y, Z¹, Z², Z³, Z⁴, Z⁵, and Z⁶ preferably contains one or more fluorine atoms.

In the above formula (I), when p is 0, X⁰ may not be present (in other words, the benzene rings on the right and left sides are directly bound to each other). When p is 1, the benzene rings on the right and left sides are bound to each other via X⁰.

Specific examples of the divalent organic group represented by X⁰ include an alkylene group, an arylene group, an aryleneoxy group, an arylenethio group, etc. Among them, an alkylene group, an aryleneoxy group, and an arylenethio group are preferred, and an alkylene group and an aryleneoxy group are more preferred, and each of these groups may be substituted with a fluorine atom. The number of carbon atoms in the above alkylene group is, for example, 1 to 12 and preferably 1 to 6.

The alkylene group substituted with a fluorine atom, which is an example of X⁰, may be, for example, -C(CF₃)₂-, -C(CF₃)₂-C(CF₃)₂-, etc. Among these examples of the alkylene group represented by X⁰, -C(CF₃)₂- is preferred.

The arylene group, which is another example of X⁰, may be, for example, any of the following groups.

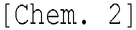

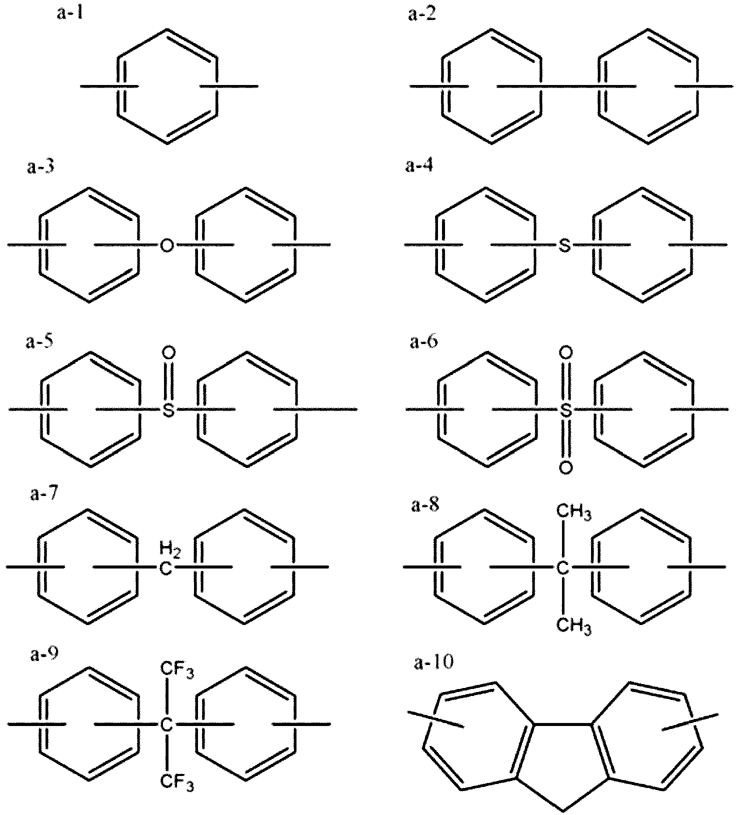

The aryleneoxy group, which is another example of X⁰, may be, for example, any of the following groups.

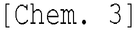

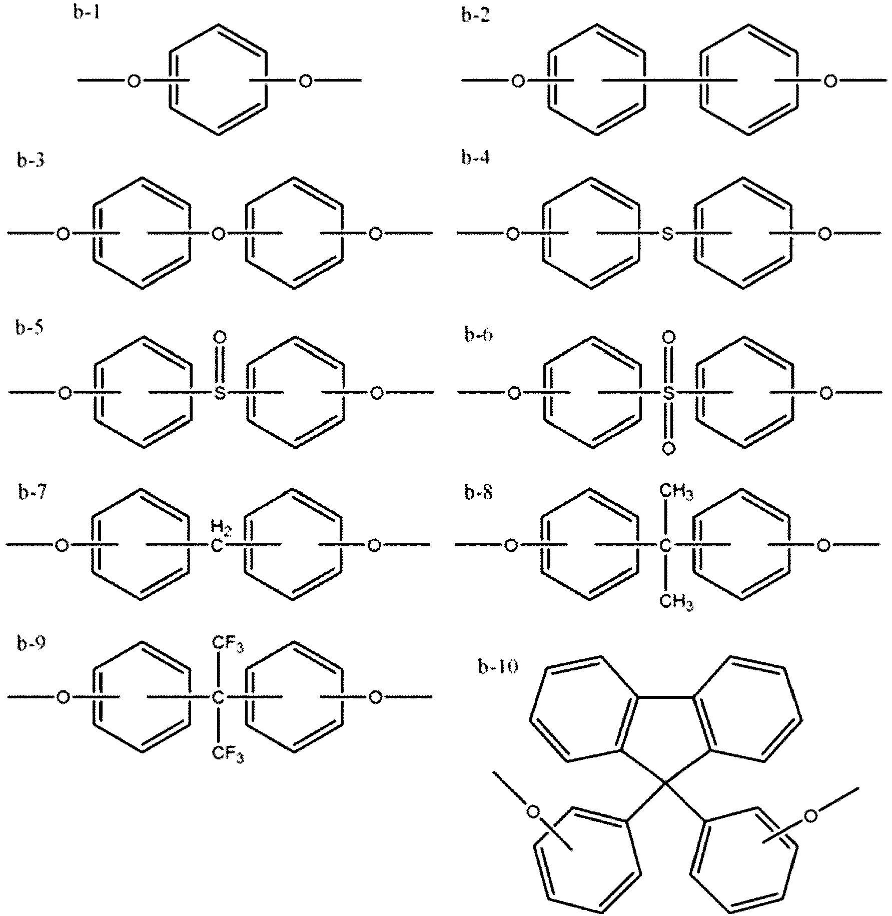

The arylenethio group, which is another example of X⁰, may be, for example, any of the following groups.

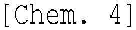

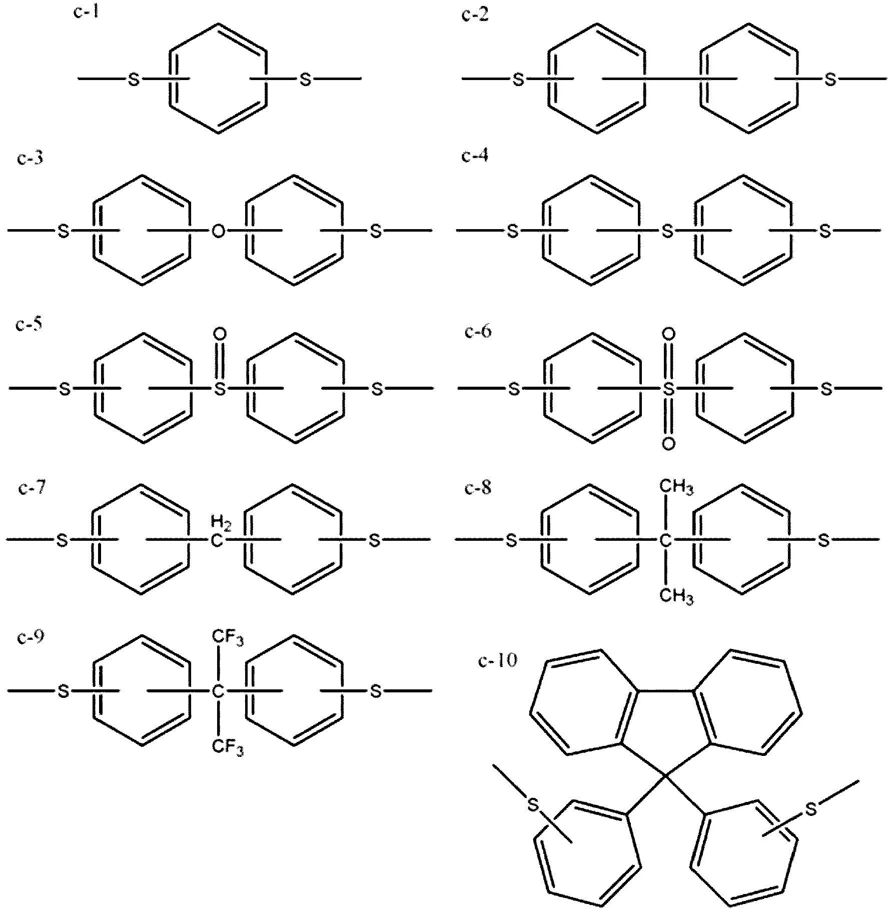

For efficient spheroid formation on a substrate, the divalent organic group represented by X⁰ is preferably selected from the group consisting of the above b-2 to b-10 and c-2 to c-10, more preferably selected from the group consisting of the above b-7 to b-9 and c-7 to c-9, and still more preferably the structure represented by b-8. Another preferable example of the divalent organic group represented by X⁰ is -C(CF₃)₂-.

The above-mentioned arylene group and aryleneoxy group, and arylenethio group, which are examples of X⁰, may be independently substituted with a group selected from the group consisting of a halogen atom (e.g., a fluorine atom, a chlorine atom, a bromine atom, or an iodine atom, preferably a fluorine atom or a chlorine atom, and more preferably a fluorine atom) , a methyl group, and a trifluoromethyl group. The number of substituents may be more than one. In this case, the substituents may be the same or different from one another. The substituent suitable for the arylene group, the aryleneoxy group, and the arylenethio group is a fluorine atom and/or a trifluoromethyl group, and preferably a fluorine atom. In the case where Y contains no fluorine atom, the arylene group, the aryleneoxy group, and the arylenethio group are preferably substituted with at least one or more fluorine atoms.

In the above formula (I), the divalent organic group represented by Y is not particularly limited and may be, for example, a divalent organic group having an aromatic ring. More specifically, the divalent organic group having an aromatic ring may be a group having one benzene ring; or a group having a structure in which two or more benzene rings are coupled directly or via a carbon atom (namely, a single bond or an alkylene group), an oxygen atom, or a sulfur atom. Specific examples include the following groups.

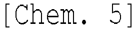

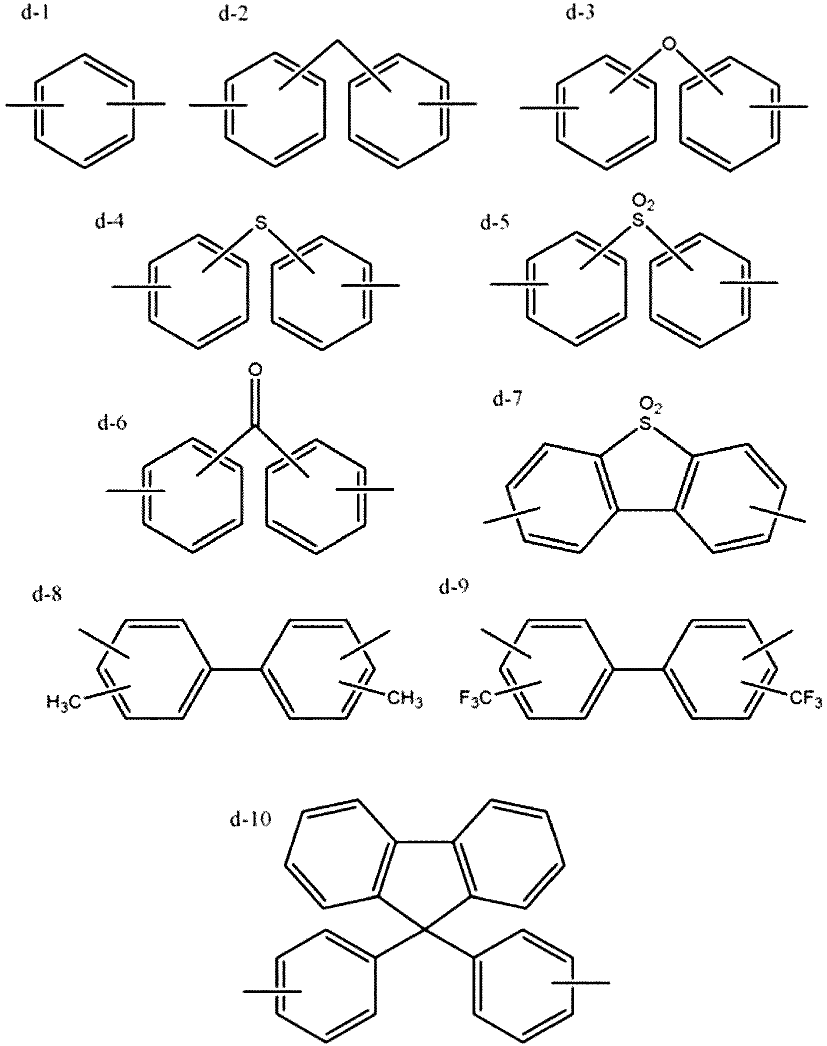

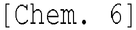

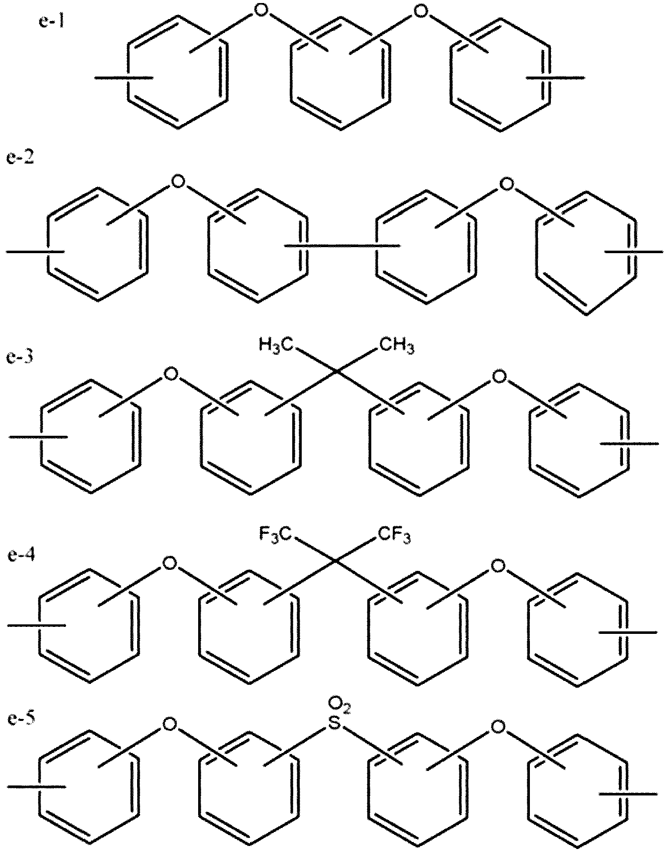

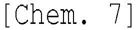

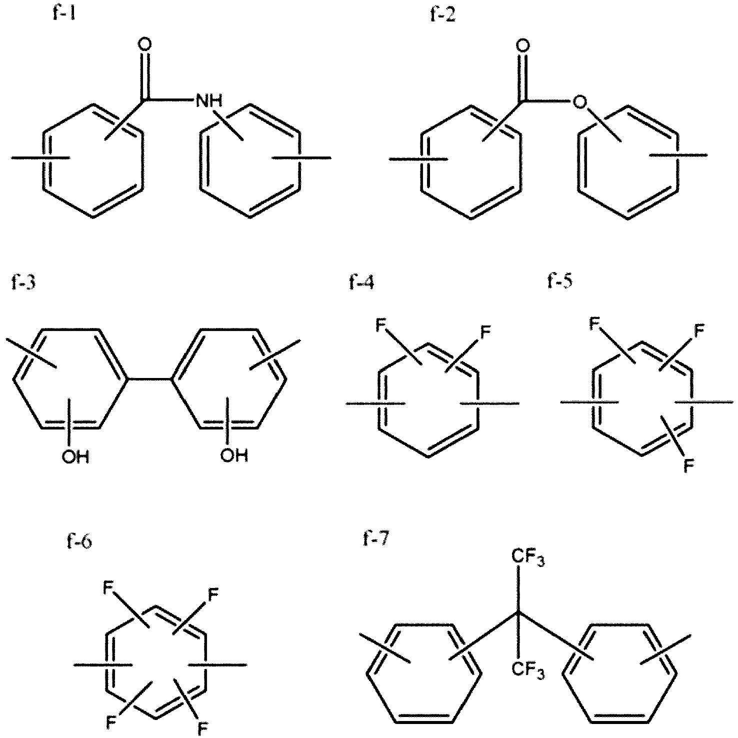

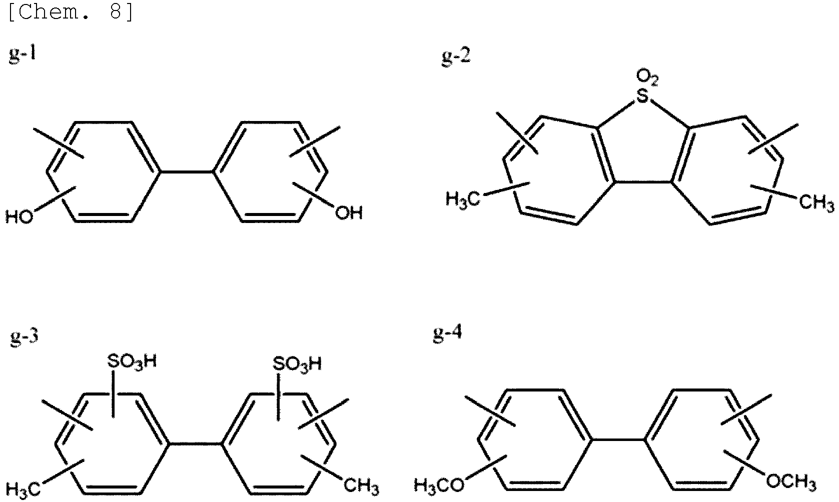

When possible, the above-mentioned divalent organic group having an aromatic ring, which is an example of Y, may be substituted with a group selected from the group consisting of a halogen atom (e.g., a fluorine atom, a chlorine atom, a bromine atom, or an iodine atom, preferably a fluorine atom or a chlorine atom, and more preferably a fluorine atom) , a methyl group, and a trifluoromethyl group. The number of substituents may be more than one. In this case, the substituents may be the same or different from one another. Particularly in the case where X⁰ contains no fluorine atom, the substituent suitable for the divalent organic group having an aromatic ring is preferably a fluorine atom and/or a trifluoromethyl group, and more preferably a fluorine atom.

In view of spheroid-forming efficiency, Y in the above formula (I) is preferably a structure selected from the group consisting of d-3, d-9, e-1 to e-4, f-6, and f-7, and more preferably a structure of e-1, e-3, or e-4.

In the above formula (I), Z¹, Z², Z³, Z⁴, Z⁵, and Z⁶ may be the same or different from one another and are independently selected from a hydrogen atom, a fluorine atom, a chlorine atom, a bromine atom, and an iodine atom. In the case where at least one of X⁰ and Y contains no fluorine atom, at least one of Z¹, Z², Z³, Z⁴, Z⁵, and Z⁶ is preferably a fluorine atom.

In view of spheroid-forming efficiency, the divalent organic group represented by X⁰ in the above formula (I) is selected from the group consisting of -C(CF₃)₂-, the above b-2 to b-10, and c-2 to c-10; and Y is selected from the group consisting of d-3, d-9, e-1 to e-4, f-6, and f-7 in one preferable embodiment of the present invention. In one more preferable embodiment of the present invention, the divalent organic group represented by X⁰ in the above formula (I) is selected from the group consisting of -C(CF₃)₂-, b-7 to b-9, and c-7 to c-9; and Y is selected from the group consisting of e-1, e-3, and e-4.

The polyimide resin having a structural unit represented by formula (I) can be obtained by calcination of a polyamic acid obtained by polymerization of an acid dianhydride and a diamine . The imidization degree of the "polyimide resin having a structural unit represented by formula (I)" does not have to be 100%. That is, the polyimide resin having a structural unit represented by formula (I) may have only a structural unit represented by the above formula (I), or alternatively may partially have a structural unit in which an amic acid remains as is and is not converted to a cyclic imide structure due to insufficient cyclodehydration, as long as the desired effects of the present invention are not impaired.

The synthesis reaction of the polyamic acid is preferably performed in an organic solvent. The organic solvent used for the synthesis reaction of the polyamic acid is not particularly limited as long as the organic solvent allows a reaction of an acid dianhydride and a diamine to proceed efficiently and is inert to these compounds. Examples of the organic solvent include polar solvents, such as N-methyl pyrrolidone (NMP), N,N-dimethylacetamide, N,N-dimethylformamide, tetrahydrofuran, dimethyl sulfoxide, sulfolane, methyl isobutyl ketone, acetonitrile, benzonitrile, nitrobenzene, nitromethane, acetone, methyl ethyl ketone, isobutyl ketone, and methanol; nonpolar solvents, such as xylene and toluene; etc. Among these examples, polar solvents are preferred. One of these organic solvents may be used alone, and also a mixture of two or more of them may be used. The reaction mixture after amidation may be directly subjected to thermal imidization. The concentration of the polyamic acid in the solution of the polyamic acid is not particularly limited and is preferably 5 wt% or more, more preferably 10 wt% or more; and is preferably 50 wt% or less, more preferably 40 wt% or less in view of the polymerization reactivity of the resin composition, its viscosity after polymerization, and its ease of handling in subsequent film production and calcination.

The polyamic acid is subjected to thermal or chemical imidization to yield a resin composition comprising a fluorine-containing polyimide. In a specific embodiment, the polyamic acid is subjected to heat treatment for imidization (thermal imidization) to yield a resin composition comprising a fluorine-containing polyimide. The polyimide obtained by thermal imidization is preferable for cell culture applications because the reaction product does not contain any residual catalyst.

In the case of thermal imidization, for example, the polyamic acid is calcinated under an air atmosphere, preferably an inert gas atmosphere, such as a nitrogen, helium, or argon atmosphere, or under vacuum, preferably at a temperature of 50 to 400°C, more preferably 100 to 380°C, preferably for 0.1 to 10 hours, more preferably 0.2 to 5 hours, to yield a resin composition comprising a polyimide.

The polyamic acid used for thermal imidization is preferably in the form of a solution in a suitable solvent. The solvent may be any solvent that can dissolve the polyamic acid. The solvent may be any of the solvents listed above for the synthesis reaction of the polyamic acid.

In the case of chemical imidization, the polyamic acid can be directly converted to an imide by a reaction using the cyclodehydration reagent described below in a suitable solvent.

The cyclodehydration reagent is not particularly limited as long as it is capable of chemically cyclodehydrating the polyamic acid into a polyimide. For efficient imidization, a tertiary amine compound alone or a combination of a tertiary amine compound and a carboxylic anhydride is preferably used as the cyclodehydration reagent.

Examples of the tertiary amine compound include trimethylamine, triethylamine, tripropylamine, tributylamine, pyridine, 1,4-diazabicyclo[2.2.2]octane (DABCO), 1,8-diazabicyclo[5.4.0]undec-7-ene, 1,5-diazabicyclo[4.3.0]non-5-ene, N,N,N',N'-tetramethyldiaminomethane, N,N,N',N'-tetramethylethylenediamine, N,N,N',N'-tetramethyl-1,3-propanediamine, N,N,N',N'-tetramethyl-1,4-phenylenediamine, N,N,N',N'-tetramethyl-1,6-hexanediamine, N,N,N',N'-tetraethylmethylenediamine, N,N,N',N'-tetraethylethylenediamine, etc. Among these examples, pyridine, DABCO, and N,N,N',N'-tetramethyldiaminomethane are preferred, and DABCO is more preferred. One kind of tertiary amine or two or more kinds of tertiary amines may be used.

Examples of the carboxylic anhydride include acetic anhydride, trifluoroacetic anhydride, propionic anhydride, butyric anhydride, isobutyric anhydride, succinic anhydride, maleic anhydride, etc. Among these examples, acetic anhydride and trifluoroacetic anhydride are preferred, and acetic anhydride is more preferred. One kind of carboxylic anhydride or two or more kinds of carboxylic anhydrides may be used.

The solvent used for dissolution of the polyamic acid in chemical imidization is preferably a polar solvent that is highly capable of dissolving the polyamic acid. Examples of such a solvent include tetrahydrofuran, N,N-dimethylacetamide, N,N-dimethylformamide, N-methyl pyrrolidone, dimethyl sulfoxide, etc. Among these examples, one or more kinds selected from the group consisting of N,N-dimethylacetamide, N,N-dimethylformamide, and N-methyl pyrrolidone are preferred in order for the reaction to proceed uniformly. In the case where these solvents are used as the solvent for amidation, the reaction mixture after amidation can be directly used for chemical imidization as it is without isolation of the polyamic acid.

The weight-average molecular weight of the polyimide resin is, for example, 5,000 to 2,000,000, preferably 8,000 to 1, 000, 000, and still more preferably 20, 000 to 500, 000. As used herein, the weight-average molecular weight of the resin is measured by the method described below. When the weight-average molecular weight is within the above range, favorable results can be obtained in terms of synthesis of the polyimide resin, its ease of handling, film formation, and spheroid-forming efficiency.

### Measurement of weight-average molecular weight

Apparatus: HCL-8220GPC, manufactured by TOSOH
Column: TSKgel Super AWM-H
Eluent (LiBr·H₂O, NMP with phosphoric acid): 0.01 mol/L Measuring method: A 0.5 wt% solution is prepared using an eluent, and the molecular weight is calculated based on a standard curve of polystyrene prepared in advance.

The cell-adhesive substance or surface [the hydrophobic (particularly, not ultra-hydrophobic) cell-adhesive substance or surface] may preferably have a static water contact angle of 70° or more and/or a sliding angle of 15° or more. It may preferably have a static water contact angle of 70° or more and a sliding angle of 15° or more.

By using the cell-adhesive substance or surface that meets these requirements, spheroid formation is further facilitated.

In view of spheroid adhesion strength and spheroid-forming efficiency, the static water contact angle is more preferably 75° or more (e.g., more than 75°), still more preferably 77° or more, still more preferably 79° or more, and yet still more preferably 80° or more (e.g., more than 80°). When the upper limit of the static water contact angle is specified, the static water contact angle is, for example, less than 150°, preferably 120° or less (e.g., less than 120°) , more preferably 110° or less, and still more preferably 100° or less (e.g., less than 99°, 98° or less, 97° or less, 95° or less, etc.) .

On the other hand, the cell-adhesive substance or surface [the hydrophilic (particularly, not ultra-hydrophilic) cell-adhesive substance] may have a static water contact angle of 65° or less, more preferably 55° or less, and still more preferably 50° or less. When the lower limit is specified, the static water contact angle may be 0° or more, more preferably 5° or more, and still more preferably 10° or more.

In view of spheroid-forming efficiency, the sliding angle may be 18° or more, 19° or more, 20° or more, 22° or more, 24° or more, 26° or more, 28° or more, or 30° or more, and among these examples, a higher sliding angle is more preferable. When the upper limit is specified, the sliding angle is, for example, less than 80°, preferably 70° or less (e.g., less than 70°) , more preferably 60° or less (e.g., less than 60°), and still more preferably 50° or less (e.g., less than 50°) . The static water contact angle and the sliding angle may be measured by the methods described below.

### Measuring method of static water contact angle

Apparatus: Automatic contact angle meter (DM-500; manufactured by Kyowa Interface Science Co., Ltd.)
Measuring method: Two microliters of water is dropped onto a surface (a non-cell adhesive surface or a cell-adhesive surface) or a film (a film formed of a non-cell adhesive substance or a cell-adhesive substance), and then immediately the contact angle of the sessile water drop is measured (measurement temperature: 25°C).

### Measuring method of sliding angle

Apparatus: Automatic contact angle meter (DM-500; manufactured by Kyowa Interface Science Co., Ltd.)
Measuring method: Twenty-five microliters of water is dropped onto a surface (a non-cell adhesive surface or a cell-adhesive surface) or a film (a film formed of a non-cell adhesive substance or a cell-adhesive substance) on the substrate, and the substrate is gradually tilted. The tilt angle at which the water drop begins to slide down is recorded as a sliding angle (measurement temperature: 25°C).

In order to enhance the homogeneity in size of spheroids and the circularity of spheroids, it is also important to optimize the balance between the cell adhesiveness of the cell-adhesive surface and that of the non-cell adhesive surface. In this context, the non-cell adhesive surface may have some level of cell adhesiveness as long as its level is lower than that of the cell-adhesive surface. For example, in the case where the static water contact angle is used as an indicator, the difference in static water contact angle (or an absolute value thereof) between the cell-adhesive surface (or the cell-adhesive substance) and the non-cell adhesive surface (or the non-cell adhesive substance) is preferably 3° or more (e.g., 5° or more), more preferably 10° or more (e.g., 12° or more), and still more preferably 15° or more. In addition, the upper limit can be determined as appropriate for the combination of hydrophobicity and hydrophilicity of the non-cell adhesive substance (surface) and the cell-adhesive substance (surface), etc. and is not particularly limited. For example, the upper limit may be 100°, 90°, 80°, 70°, 60°, 50°, 40°, 30°, etc.

The resin which constitutes the cell-adhesive surface may further comprise an additive component, such as a plasticizer and an antioxidant.

The proportion of the cell-adhesive surface of the bottom face of the recess is not particularly limited and is preferably 90% or more, 95% or more, 99% or more, or substantially 100% of the area of the bottom face of the recess.

In the cell culture sheet of the present invention, the structure of the recess is as described above, and the non-recessed surface, i.e., the sheet surface, preferably has a non-cell adhesive surface in order to simplify the production of the cell culture sheet. When the non-recessed surface has such a constitution, cells are guided to form a spheroid in the recess once seeded on the non-recessed surface. For example, in Fig. 1, the non-recessed surface is represented as the sheet surface 12. The non-cell adhesive surface of the sheet surface may be the same as or different from the non-cell adhesive surface of the inner circumferential face of the recess. In the case where the non-cell adhesive surface of the sheet surface is the same as the non-cell adhesive surface of the inner circumferential face of the recess, the sheet surface and the inner circumferential faces of the recesses has a continuous surface. The proportion of the non-cell adhesive surface in the sheet surface (the non-recessed surface) is not particularly limited and is preferably 90% or more, 95% or more, 99% or more, or substantially 100% of the area of the non-recessed surface. The proportion of the non-cell adhesive surface in the combined total area of the non-recessed surface and the inner circumferential faces of the recesses is not particularly limited and is preferably 90% or more, 95% or more, 99% or more, or substantially 100% of the combined total area of the non-recessed surface and the inner circumferential faces of the recesses.

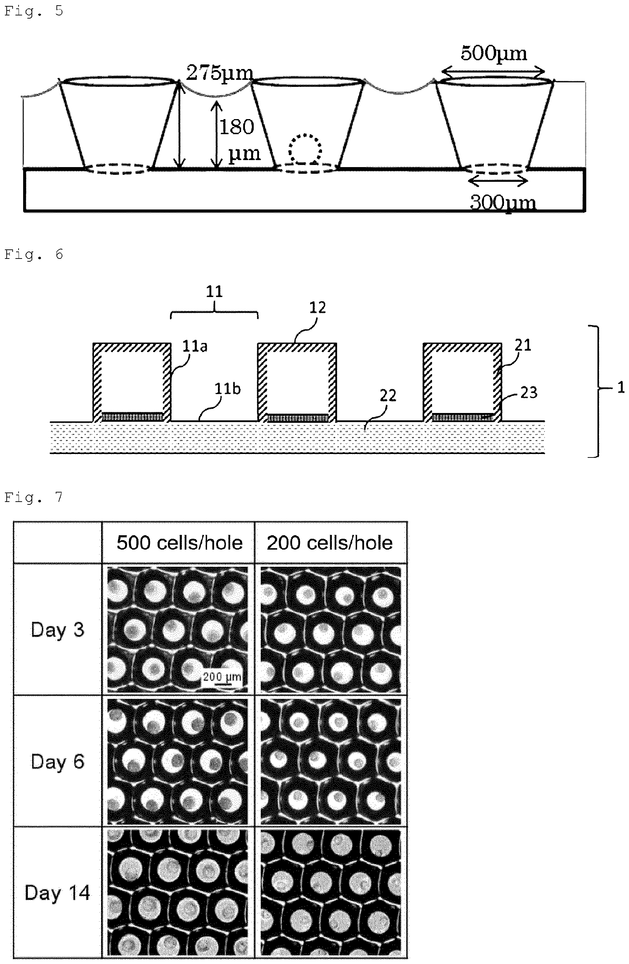

For reference, schematic views of a cell cultured in one recess of the cell culture sheet are shown in Figs. 4 and 5. The cell culture sheet of the present invention, which comprises a plurality of recesses, can also be referred to as a layered structure comprising a layer including the bottom faces of the recesses and a layer including the inner circumferential faces of the recesses, as seen from Figs. 4 and 5. The layer including the inner circumferential faces of the recesses is a layer having through-holes. Therefore, in one embodiment, the cell culture sheet of the present invention is a laminate comprising a layer having the non-cell adhesive surface and a layer having the cell-adhesive surface, wherein the layer having the non-cell adhesive surface has through-holes.

The layer having the non-cell adhesive surface may be a layer of a non-cell adhesive substance immobilized on a layered substrate or alternatively may be a layer of a non-cell adhesive substance. In order to facilitate through-hole formation and simplify the production of the cell culture sheet, the layer having the non-cell adhesive surface is preferably a layer of a non-cell adhesive substance immobilized on a layered substrate. In this case, the cell culture sheet can be produced more simply. In addition, when the cell culture sheet is produced by, for example, forming through-holes or recesses in the substrate, followed by immobilizing a non-cell adhesive substance, there is no risk of damaging the non-cell adhesive surface during recess formation in the production of the cell culture sheet, leading to enhancement in the cell culture performance of the cell culture sheet.

The layered substrate may be any layered substrate known in the technical field. Examples of the layered substrate include plates formed of synthetic resins, such as polystyrene, polyethylene, polypropylene, polycarbonate, polyamide, polyacetal, polyester (polyethylene terephthalate etc.), polyurethane, polysulfone, polyacrylate, polymethacrylate, polyvinyl, polycycloolefin, polyether ketone, polyether ether ketone, polyimide, and silicone; synthetic rubbers, such as EPDM (ethylene propylene diene monomer), and natural rubbers; glasses; ceramics; metallic materials, such as stainless steel; etc. In addition, a preferable embodiment of the substrate is a transparent substrate.

The immobilization of the non-cell adhesive substance on the layered substrate can be performed in the same manner as described above for the immobilization of the non-cell adhesive substance at the inner circumferential face of the recess. In view of workability, the immobilization step preferably follows the step of through-hole formation, which is described later.

The layer having the non-cell adhesive surface preferably includes the sheet surface and through-holes of the cell culture sheet of the present invention. The wall of the through-hole preferably corresponds to the inner circumferential face of the recess described above. The sizes and shapes of the opening and the opposite end of the through-hole can be determined in the same manner as described above for the recess. The depth of the through-hole corresponds to the thickness of the layer having the non-cell adhesive surface and also to the depth of the recess described above. The depth of the through-hole can be determined in the same manner as described above for the recess. In the case where the non-cell adhesive substance is immobilized on the layered substrate, the layer of the non-cell adhesive substance has a thickness of, for example, preferably 1 nm or more, more preferably 10 nm or more. As long as the thickness of the whole layer including the layer of the non-cell adhesive substance and the substrate is within the above-mentioned range of the thickness of the layer having the non-cell adhesive surface, an appropriate thickness of the layer of the non-cell adhesive substance can be selected.

The method for through-hole formation is not particularly limited and may be any method that enables through-holes of the above-specified size to be formed. For example, piercing (drills etc.), optical microprocessing (lasers (e.g., a CO₂ laser, an excimer laser, a semiconductor laser, a YAG laser) etc.), etching, embossing, and other processing techniques can be used for through-hole formation. Tapered through-holes may be formed by such processing techniques. In this case, the surrounding area of the end of the through-hole may be deformed, that is, the thickness of the layer at the periphery of the opening may be different from that at the midpoint between adjacent openings, for example, as shown in Fig. 5.

The layer having the cell-adhesive surface may be a layer of a cell-adhesive substance immobilized on a layered substrate or alternatively may be a layer of a cell-adhesive substance.

The thickness of the layer having the cell-adhesive surface is, for example, 1 to 4 mm and preferably 1 µm to 1 mm. The thickness of the layer having the cell-adhesive surface may be the same as that of the bottom face of the recess.

In one embodiment, the cell culture sheet of the present invention further comprises an adhesive layer between the layer having the cell-adhesive surface and the layer having the non-cell adhesive surface. For example, in one embodiment shown in Fig. 6, the cell culture sheet comprises an adhesive layer 23 between a layer of a cell-adhesive substance 22 and a portion having a non-cell adhesive substance 21 superficially immobilized.

The adhesive layer may be any known adhesive layer in the technical field. Examples of the adhesive layer include adhesive layers made of silicone-based resins, synthetic rubbers, natural rubbers, etc. Preferred are low-dissolution adhesive layers. Commercial double-sided tapes etc. are also preferable.

The thickness of the adhesive layer is not particularly limited, and an appropriate thickness can be selected as long as the effects of the present invention are not impaired. For example, the thickness may be 0.5 to 100 µm.

The cell culture sheet of the present invention may further comprise a layer other than the layers described above. A cavity-containing layer may be comprised in the cell culture sheet of the present invention.

The thickness of the cell culture sheet of the present invention is not particularly limited and is preferably 10 to 5000 µm, more preferably 100 to 2000 µm in view of ease of handling. The area (dimension) of the cell culture sheet of the present invention is not particularly limited and is, for example, 0.01 to 10000 cm², preferably 0.03 to 5000 cm².

The cell culture sheet of the present invention can be directly placed in a known cell culture device. Before placed in a culture device, the cell culture sheet may be processed for size adjustment as appropriate for the size of the device.

The cells suitable for use on the cell culture sheet of the present invention are not limited. For example, when the cell culture sheet of the present invention is used for culturing a type of cells that can bind together to form cell aggregates, the cells can be cultured in an adherent state on the cell-adhesive surface of the bottom face of the recess. This facilitates medium change, resulting in the reduction in cell loss at the time of medium change. Thus, spheroids can easily be prepared. The cells may be derived from any animal, organ, or tissue, and an appropriate type of cells for each individual purpose can be selected.

Specific examples of the cells that can be used include primary cells and established cell lines derived from organs and tissues (brain, liver, pancreas, spleen, heart, lung, intestine, cartilage, bone, fat, kidney, nerve, skin, bone marrow, embryo, etc.) of humans and non-human animals (monkeys, pigs, dogs, rats, mice, etc.) and also include genetic engineered cells derived from the foregoing cells.

More specific examples include ES cells, iPS cells, neural stem cells, mesenchymal stem cells, tissue stem cell (somatic stem cells), hematopoietic stem cells, cancer stem cells, and other undifferentiated stem cells and progenitor cells. Also included are differentiated cells such as gastrointestinal cells such as hepatic and pancreatic cells; kidney cells; neural cells; cardiovascular cells such as cardiomyocytes; fibroblasts derived from connective tissues such as adipocytes and skin dermis; epithelial cells of epithelial tissues such as skin epidermis; bone cells; cartilaginous cells; cells of ocular tissues such as retina; vascular cells; hematopoietic cells; germ cells; etc. Also included are malignantly transformed cells. One of these cells may be used alone, or alternatively two or more of them may be used in a mixture at any ratio.

The method for producing the cell culture sheet of the present invention is not particularly limited as long as the cell culture sheet having the structure described above can be obtained. In view of production efficiency, the production method preferably comprises laminating of a layer having a non-cell adhesive surface and a layer having a cell-adhesive surface in this order, wherein the layer having a non-cell adhesive surface has a plurality of through-holes of 1000 µm or less in diameter.

The preparation of the layer having a cell-adhesive surface and the layer having a non-cell adhesive surface can be performed according to the procedure described above for the cell culture sheet of the present invention. Similarly, the formation of the through-holes in the layer having a non-cell adhesive surface can be performed according to the procedure described above for the cell culture sheet of the present invention.

For laminating of the two layers, both layers may be separately prepared in advance and laminated to each other, or alternatively, a layer may be formed on the other layer prepared in advance. Alternatively, these methods may be employed in combination. More specifically, for example, on a sheet having a release coated surface (e.g., an organic polymer film such as a polyethylene substrate, ceramic, metal, etc.), a cell-adhesive substance is applied at an appropriate thickness by casting, spin coating, roll coating, or other techniques, and then heated to form a sheet-like layer having a cell-adhesive surface. On the other hand, for advance preparation of the layer having a non-cell adhesive surface, through-holes are formed in a substrate for the layer having a non-cell adhesive surface, and a non-cell adhesive substance is applied to coat the surface. Subsequently, after removal of the release sheet of the layer having a cell-adhesive surface, the layer having a non-cell adhesive surface prepared separately is laminated to the layer having a cell-adhesive surface to produce the above-described laminate. For laminating of the layer having a non-cell adhesive surface and the layer having a cell-adhesive surface, the adhesive layer described above may be used, or alternatively, welding (high frequency welding, ultrasonic welding, etc.) or compression bonding (thermocompression bonding etc.) may be employed.

When the cell culture sheet of the present invention is used for cell culture, cell-containing medium may be added on the surface on one side of the sheet. Alternatively, the cell culture sheet can be placed and fixed in a cell culture vessel, such as a culture dish, a flask, a culture bag, and other various types of cell culture vessels, and to the culture vessel, cell-containing medium may be added. Therefore, the present invention provides a cell culture tool comprising the cell culture sheet of the present invention.

The cell culture tool of the present invention may be in the form of a culture plate such as a single-well or multi-well plate or in the form of a cell culture vessel such as a culture Petri dish, a culture dish, a flask, or a culture bag.

The present invention also provides a method for culturing spheroids, the method comprising culturing cells using the cell culture sheet or cell culture tool of the present invention.

The medium and conditions for cell culture can be determined as appropriate for the cells to be used. If needed, the cell culture sheet or cell culture tool of the present invention is preferably subjected to degassing before use. The degassing is not particularly limited, and examples include spraying, pipetting, shaking, thermal change such as heating and cooling, centrifugation, vacuum deaeration, ultrasonication, and other commonly used treatments. Preferred are spraying, pipetting, and thermal change.

By using the cell culture sheet of the present invention, seeded cells are sorted into compartments (a plurality of recesses) of the cell culture sheet and the moderate cell adhesiveness of the substrate works beneficially, which leads to more homogeneous production of spheroids. In addition, due to the moderate cell adhesiveness, the cell culture sheet is easy to handle during culture operation, and only agitation of the cell culture sheet or cell culture tool or gentle pipetting is enough to harvest spheroids. The recesses of the cell culture sheet of the present invention are quite small with an opening of 1000 µm or less in diameter, but each recess presumably provides a distinctive cell-adhesive environment formed of its cell-adhesive bottom face and circumferential non-cell adhesive side wall, which leads to high performance of the cell culture sheet in terms of the homogeneity in size of spheroids and the circularity of spheroids, and even the yield of spheroids. However, the present invention is not bound by this presumption.

The diameter of the spheroid is not particularly limited and is, for example, 10 to 1000 µm, preferably 10 to 800 µm. The diameter of the spheroid can be measured by the usual method (e.g., using an image-analysis software, a particle size analyzer, etc.) and can be expressed as, for example, a fluid diameter or an equivalent circle diameter. The circularity of the spheroid is, for example, 0.5 to 1.0 and preferably 0.7 to 1.0.

### EXAMPLES

Hereinafter, the present invention will be described in detail by examples, but the present invention is not limited thereto. In the following Examples, "room temperature" refers to 20 to 30°C.

### Example 1: Cell culture sheet

### Preparation of layer having a cell-adhesive surface (preparation of fluorine-containing polyimide film)

2.976 g (10.2 mmol) of 1,4-bis(aminophenoxy)benzene, 4.524 g (10.2 mmol) of 4,4'-(hexafluoroisopropylidene)diphthalic anhydride, and 42.5 g of N-methyl pyrrolidone were fed into a 100-mL three-necked flask. The mixture was stirred under a nitrogen atmosphere at room temperature and then left to stand for 5 days. Thus, a fluorine-containing polyamic acid resin composition (solid content: 15.0% by mass, 6FDA/TPEQ polyamic acid) was obtained. The weight-average molecular weight of the polyamic acid was 120,000, and the viscosity was 6 Pa·s. The weight-average molecular weight of a polyamic acid and the weight-average molecular weight of a fluorine-containing polyimide after calcination are substantially the same.

The fluorine-containing polyamic acid resin composition obtained above was applied on a glass substrate using a die coater such that the thickness of the fluorine-containing polyimide film after calcination would be 40 µm. Subsequently, the coating was calcinated under a nitrogen atmosphere at 360°C for 1 hour. The calcined coating was separated from the glass substrate. Thus, a fluorine-containing polyimide film was obtained. The static water contact angle of the fluorine-containing polyimide film was 83.0°, and the sliding angle was 24.0°.

The methods for measuring the physical properties mentioned above are as follows.

### Measurement of weight-average molecular weight

Apparatus: HCL-8220GPC, manufactured by TOSOH
Column: TSKgel Super AWM-H
Eluent (LiBr·H₂O, NMP with phosphoric acid): 0.01 mol/L Measuring method: A 0.5 wt% solution is prepared using an eluent, and the molecular weight is calculated based on a standard curve of polystyrene prepared in advance.

### Measurement of viscosity

Apparatus: VISCOMETER TV-22, manufactured by AS ONE Corporation Setting: VI RANGE:H ROTOR No. 6 SPEED: 10 rpm
Standard liquids for calibrating viscometers: Nippon Grease JS14000
Measuring method: The viscosity is measured using 0.3 g of varnish after calibration with standard liquids for calibrating viscometers (measurement temperature: 23°C).

### Measurement of static water contact angle

Apparatus: Automatic contact angle meter (DM-500; manufactured by Kyowa Interface Science Co., Ltd.)
Measuring method: Two microliters of water is dropped onto a film, and then immediately the contact angle of the sessile water drop is measured (measurement temperature: 25°C).

### Measurement of sliding angle

Apparatus: Automatic contact angle meter (DM-500; manufactured by Kyowa Interface Science Co., Ltd.)
Measuring method: Twenty-five microliters of water is dropped onto a film on the substrate, and the substrate is gradually tilted. The tilt angle at which the water drop begins to slide down is recorded as a sliding angle (measurement temperature: 25°C) .

### Preparation of layer having a non-cell adhesive surface

A release tape on one side of a double-sided tape (25-µm-thick) was removed, and a transparent PET film (250 µm in thickness) was laminated to the double-sided tape. On this laminate, through-holes of 300 µm in diameter were formed in a staggered configuration with a pitch of 500 µm using a CO₂ laser (formed through-holes: 400 holes/cm², 24000 holes/sheet, hole diameter on laser incidence side: 500 µm, hole diameter on a laser emission side: 300 µm) . After that, on the surface of the PET film side, an MPC polymer solution (a 0.5% solution of a hydrophobic MPC polymer in ethanol) was applied using a spin coater (manufactured by MIKASA CO., LTD.: MS-A150) (spinning conditions: 1000 rpm for 10 seconds) such that the final thickness would be 0.05 µm and dried in a dryer at 50°C for 2 hours. Thus, a layer having a non-cell adhesive surface (static water contact angle of the coating layer on the PET film (MPC polymer coating layer): 107.5°) was obtained.

### Preparation of cell culture sheet and culture vessel

Next, the release tape on the opposite side of the double-sided tape from the non-cell adhesive surface was removed, and the layer having a cell-adhesive surface prepared above was laminated to the double-sided tape to prepare a cell culture sheet (sheet thickness: 315 µm) . The obtained cell culture sheet was placed in a culture plate. Thus, a vessel used for cell culture was finally made.

### Test Example 1

The cells used were human adipose-derived stem cells (AdSCs) . The AdSCs used were a commercial product from Lonza (trade name: PT-5006) .

### Expansion culture of cells

Frozen cells were thawed in a constant temperature water bath at 37°C and added to 9 mL of KBM ADSC-2 medium (basal medium; manufactured by Kohjin-Bio Co., Ltd.) containing 5% FBS and 1% antibiotics. After centrifugation at 500 × g for 5 minutes, the supernatant was removed, and the cells were suspended in 10 mL of the basal medium. To an 800-mL cell culture flask (manufactured by Sumitomo Bakelite Co., Ltd.) , the basal medium was added in such a volume that the total volume after addition of the cell suspension would be 30 mL. The cell suspension was added to the flask at 1.0 × 10⁶ cells/flask, and culture (expansion culture) was performed in an incubator with 5% (v/v) CO₂ at 37°C.

### Degassing

The culture vessel of Example 1 was degassed. More specifically, about 25 mL of PBS was added to the vessel, and pipetting was repeated twice for degassing. Subsequently, 12 mL of KBM ADSC-2 medium containing 1% antibiotics was added to the vessel, which was then placed in an incubator with 5% (v/v) CO₂ at 37°C overnight.

### Culture of spheroids

The medium was removed from the culture flask, and 5 mL of a cell detachment solution, Accutase (manufactured by PromoCell), was added. The flask was left to stand in an incubator with 5% (v/v) CO₂ at 37°C for about 5 minutes for cell detachment. The cell detachment solution in the flask was recovered, made up to a total volume of 25 mL with PBS, and transferred to a tube. After centrifugation at 500 × g for 5 minutes, the cells were suspended in 10 mL of KBM ADSC-2 medium containing 1% antibiotics and counted. After that, the cell suspension was adjusted to 1.0 × 10⁶ cells/mL.

The medium in the culture vessel, which had been degassed in an incubator with 5% (v/v) CO₂ at 37°C overnight, was removed, and the cells were seeded at 500 cells/well or 200 cells/well in the culture vessel. The culture vessel was left to stand in a biosafety cabinet for 15 minutes and placed in an incubator with 5% (v/v) CO₂ at 37°C for 4 hours. Subsequently, the culture vessel was taken out from the incubator, and KBM ADSC-2 medium containing 1% antibiotics was additionally added. The culture vessel was returned to the incubator with 5% (v/v) CO₂ at 37°C, and culture was started (day 0 of culture). The culture was continued until day 14. The medium was fully changed to a fresh KBM ADSC-2 medium containing 1% antibiotics every 3 days. Even when the culture vessel was agitated at the time of medium change etc., spheroids did not accidentally jump out of the culture vessel. The spheroids were collected by pipetting.

### Morphological evaluation of spheroids

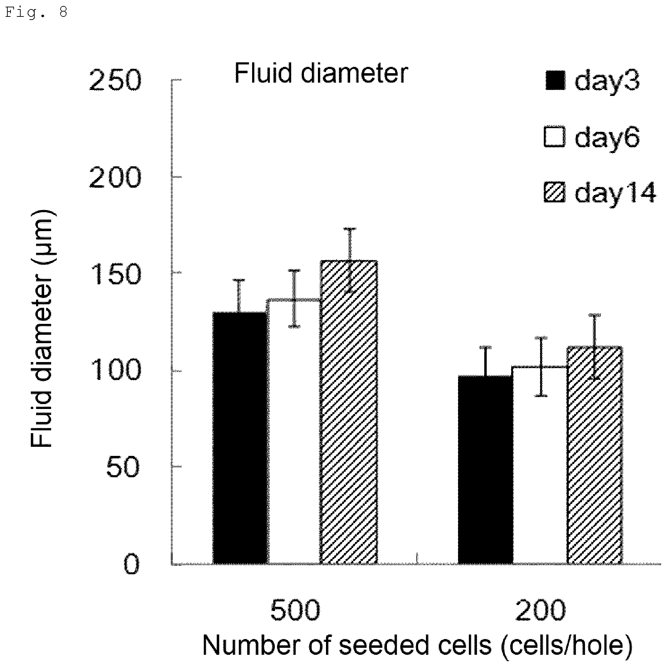

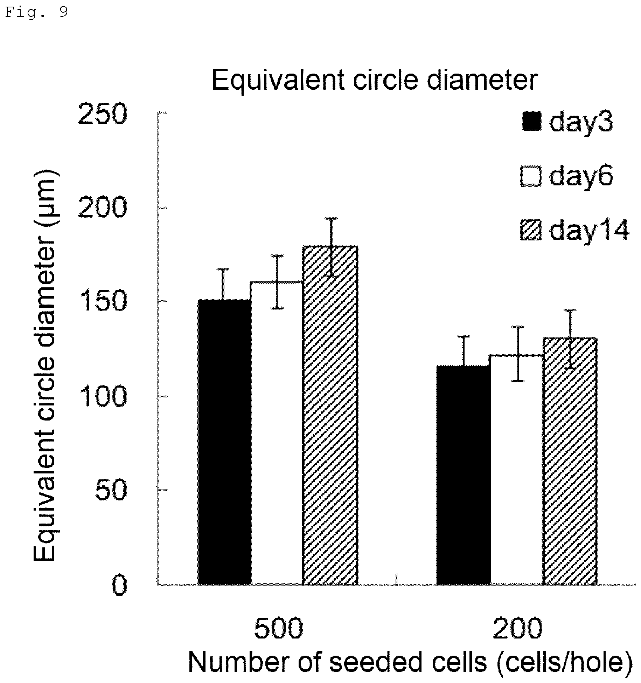

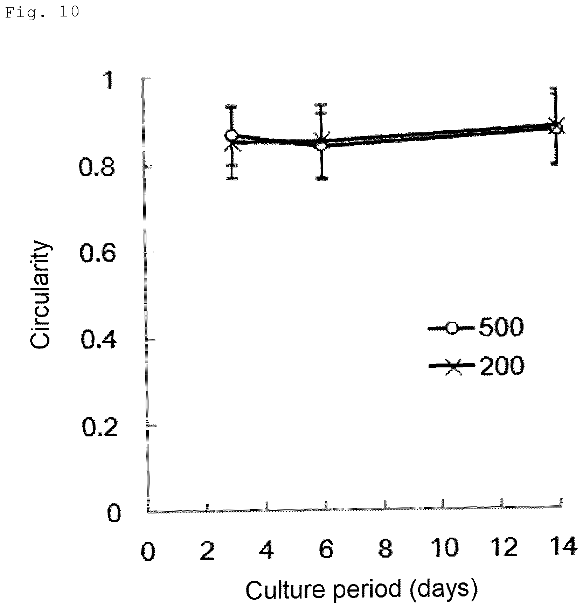

The spheroids were photographed on days 3, 6, and 14 of culture (Fig. 7) and analyzed in terms of equivalent circle diameter, fluid diameter, and circularity using an image-analysis software WinROOF (manufactured by MITANI CORPORATION) (Figs. 8 to 10).

The size of the obtained spheroids was highly homogeneous (500 cells/well: equivalent circle diameter: 151 to 179 µm, SD: 14 to 16 µm; and 200 cells/well: equivalent circle diameter: 116 to 130 µm, SD: 14 to 15 µm) . The circularity of the obtained spheroids was high (500 cells/well: circularity: 0.841 to 0.873, SD: 0.067 to 0.083; and 200 cells/well: circularity: 0.849 to 0.882, SD: 0.080 to 0.086). The data also show that the different numbers of seeded cells resulted in the formation of different-sized spheroids. The above results demonstrate that a large quantity of spheroids can be homogeneously obtained in a stable and easy manner using the cell culture sheet of the present invention.

### Test Example 2

A human bone marrow-derived mesenchymal stem cell line (UE7T-13 cells; JCRB1154) was cultured using a cell culture sheet which had been prepared and degassed as described in Example 1. The medium used was DMEM + 10% FBS.

More specifically, 4.8 × 10⁶ cells were seeded on the cell culture sheet (200 cells per well) in 22 mL of medium and cultured in an incubator with 5% (v/v) CO₂ at 37°C for 3 days. The medium was not changed during culture . The morphology of the spheroids was evaluated on days 3 and 4 of culture.

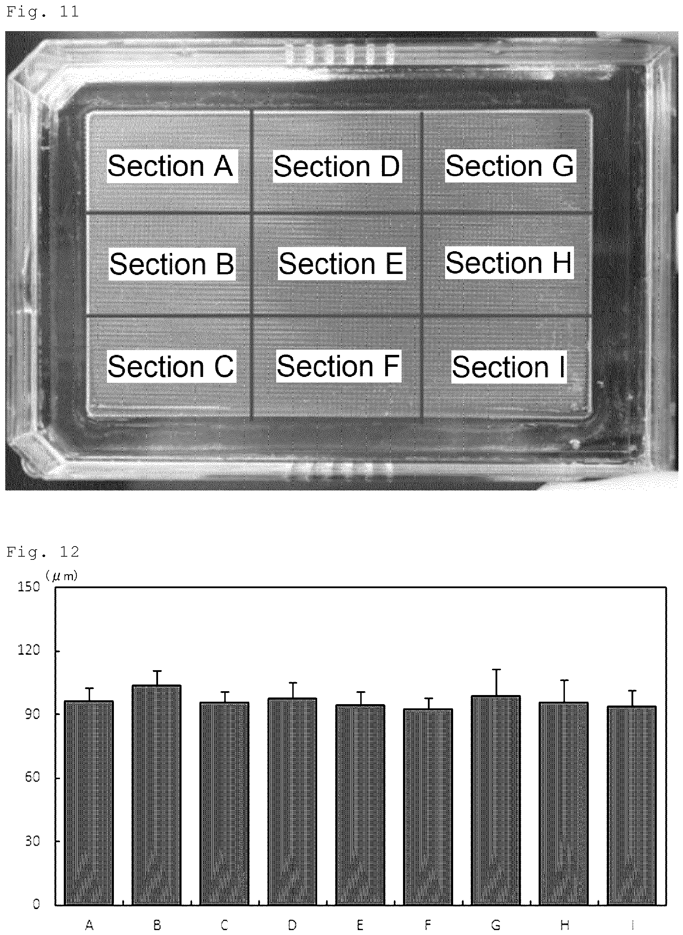

For morphological evaluation, the cell culture sheet was divided into 9 sections (sections A to I) (Fig. 11), the spheroids in each section were photographed and analyzed in terms of equivalent circle diameter using an image-analysis software WinROOF (manufactured by MITANI CORPORATION) (Fig. 12 shows the analysis results for day 3 of culture).

The size of the obtained spheroids was highly homogeneous (day 3 of culture: equivalent circle diameter: 96 µm, SD: 8 µm; and day 4 of culture: equivalent circle diameter: 97 µm, SD 9 µm) and did not vary depending on the section geometry.

### Test Example 3

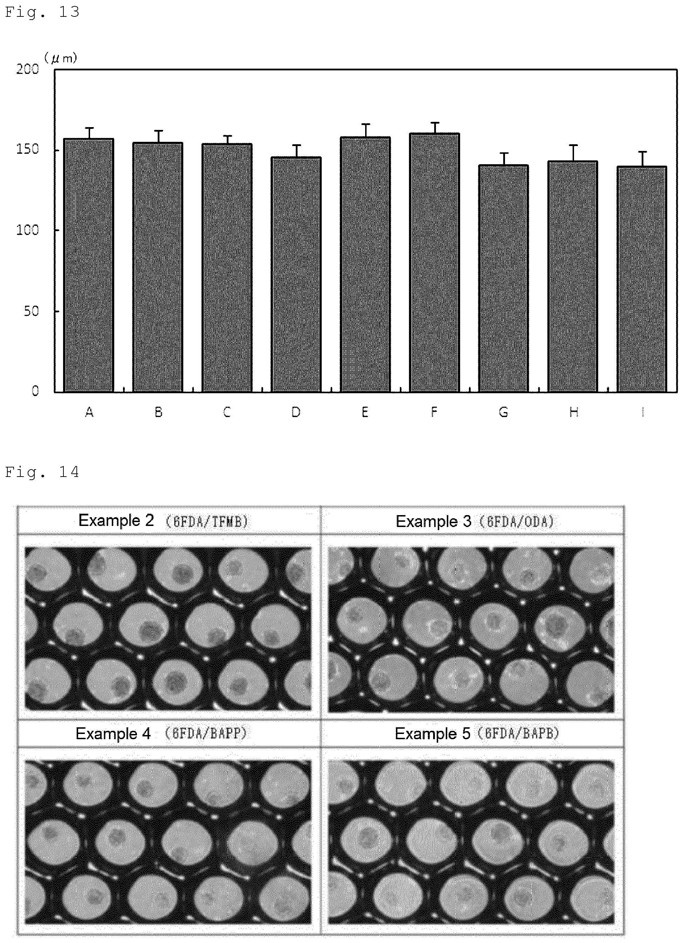

Cell culture was performed in the same manner as described in Test Example 2 except that the number of cells seeded per cell culture sheet was 1.2 × 10⁷ cells (500 cells per well). The equivalent circle diameter of the spheroids was evaluated on day 1 of culture (Fig. 13).

The size of the obtained spheroids was highly homogeneous (day 1 of culture: equivalent circle diameter: 151 µm, SD: 11 µm) and did not vary depending on the section geometry.

### Examples 2 to 5

Cell culture vessels were prepared in the same manner as described in Example 1 except that the preparation of the layer having a cell-adhesive surface (preparation of a fluorine-containing polyimide film) was performed as described below. In each Example, the weight-average molecular weight of a polyamic acid and the weight-average molecular weight of a fluorine-containing polyimide after calcination are substantially the same.

### Example 2: 6FDA/TFMB

21.792 g (0.049 mol) of 4,4'-(hexafluoroisopropylidene)diphthalic anhydride and 148.75 g of N-methyl pyrrolidone were fed into a 500-mL three-necked flask to prepare a solution. To this, a solution of 15.708 g (0.049 mol) of 2,2'-bis(trifluoromethyl)benzidine in 63.75 g of N-methyl pyrrolidone was added dropwise. The mixture was stirred under a nitrogen atmosphere at room temperature and then left to stand for 5 days. Thus, a fluorine-containing polyamic acid resin composition (solid content: 15% by mass, 6FDA/TFMB polyamic acid) was obtained. The weight-average molecular weight of the polyamic acid was 189,000, and the viscosity was 9.1 Pa·s. The same procedure as described in Example 1 was performed using the obtained fluorine-containing polyamic acid resin composition to give a fluorine-containing polyimide film. The static water contact angle of the fluorine-containing polyimide film was 82.1°, and the sliding angle was 19.9°.

### Example 3: 6FDA/ODA

26.89 g (0.058 mol) of 4,4'-(hexafluoroisopropylidene)diphthalic anhydride and 154.7 g of N-methyl pyrrolidone were fed into a 500-mL three-necked flask to prepare a solution. To this, a solution of 12.11 g (0.058 mol) of 4,4'-diaminodiphenyl ether in 66.3 g of N-methyl pyrrolidone was added dropwise. The mixture was stirred under a nitrogen atmosphere at room temperature and then left to stand for 5 days. Thus, a fluorine-containing polyamic acid resin composition (solid content: 15% by mass, 6FDA/ODA polyamic acid) was obtained. The weight-average molecular weight of the polyamic acid was 153, 000, and the viscosity was 7.7 Pa·s. The same procedure as described in Example 1 was performed using the obtained fluorine-containing polyamic acid resin composition to give a fluorine-containing polyimide film. The static water contact angle of the fluorine-containing polyimide film was 79.5°, and the sliding angle was 31.0°.

### Example 4: 6FDA/BAPP

19.49 g (0.044 mol) of 4,4'-(hexafluoroisopropylidene)diphthalic anhydride and 148.75 g of N-methyl pyrrolidone were fed into a 500-mL three-necked flask to prepare a solution. To this, a solution of 18.01 g (0.044 mol) of 2,2-bis(4-(4-aminophenoxy)phenyl)propane in 63.75 g of N-methyl pyrrolidone was added dropwise. The mixture was stirred under a nitrogen atmosphere at room temperature and then left to stand for 5 days. Thus, a fluorine-containing polyamic acid resin composition (solid content: 15% by mass, 6FDA/BAPP polyamic acid) was obtained. The weight-average molecular weight of the polyamic acid was 185, 000, and the viscosity was 7.1 Pa·s. The same procedure as described in Example 1 was performed using the obtained fluorine-containing polyamic acid resin composition to give a fluorine-containing polyimide film. The static water contact angle of the fluorine-containing polyimide film was 82.5°, and the sliding angle was 32.2°.

### Example 5: 6FDA/BAPB

20.50 g (0.046 mol) of 4,4'-(hexafluoroisopropylidene)diphthalic anhydride and 148.75 g of N-methyl pyrrolidone were fed into a 500-mL three-necked flask to prepare a solution. To this, a solution of 17.00 g (0.046 mol) of 4,4'-bis(4-aminophenoxy)biphenyl in 63. 75 g of N-methyl pyrrolidone was added dropwise. The mixture was stirred under a nitrogen atmosphere at room temperature and then left to stand for 5 days. Thus, a fluorine-containing polyamic acid resin composition (solid content: 15% by mass, 6FDA/BAPB polyamic acid) was obtained. The weight-average molecular weight of the polyamic acid was 112,000, and the viscosity was 8.1 Pa·s. The same procedure as described in Example 1 was performed using the obtained fluorine-containing polyamic acid resin composition to give a fluorine-containing polyimide film. The static water contact angle of the fluorine-containing polyimide film was 79.0°, and the sliding angle was 32.2°.

### Test Example 4

The cells used in this Example were the same as those used in Test Example 1.

### Expansion culture of human adipose-derived stem cells

Frozen cells were thawed in a constant temperature water bath at 37°C and added to 9 mL of KBM ADSC-2 medium (basal medium; manufactured by Kohjin-Bio Co., Ltd.) containing 5% FBS and 1% antibiotics. After centrifugation at 210 × g for 5 minutes, the supernatant was removed, and the cells were suspended in 1 mL of the basal medium. To an 800-mL cell culture flask (manufactured by Sumitomo Bakelite Co., Ltd.) , the basal medium was added in such a volume that the total volume after addition of the cell suspension would be 30 mL. The cell suspension was added to the flask at 1.0 × 10⁶ cells/flask, and culture was performed in an incubator with 5% (v/v) CO₂ at 37°C.

### Degassing of vessels

Culture vessels of Example 2 to 5 were degassed. More specifically, about 2 mL of PBS was added to each vessel, and pipetting was repeated twice for degassing. Subsequently, 0.2 mL of KBM ADSC-2 medium containing 1% antibiotics was added to each vessel, which was then placed in an incubator with 5% (v/v) CO₂ at 37°C overnight.

### Culture of spheroids

The medium was removed from the culture flask, and 5 mL of a cell detachment solution, Accutase (manufactured by PromoCell), was added. The flask was left to stand in an incubator with 5% (v/v) CO₂ at 37°C for about 5 minutes for cell detachment. The cell detachment solution in the flask was recovered, made up to a total volume of 15 mL with PBS, and transferred to a tube. After centrifugation at 210 × g for 5 minutes, the cells were suspended in 2 mL of KBM ADSC-2 medium containing 1% antibiotics and counted. After that, the cell suspension was adjusted to 1.0 × 10⁶ cells/mL.

The medium in each culture vessel, which had been degassed in an incubator with 5% (v/v) CO₂ at 37°C overnight, was removed, and the cells were seeded at 500 cells/well in each culture vessel. After that, culture was started as described in Test Example 1 and continued until day 3. The obtained spheroids were photographed as described in Test Example 1 for morphological evaluation. The results are shown in Fig. 14.

The results show that spheroids were successfully formed even when different types of resins of the cell-adhesive surface and different types of cell lines were used. The homogeneity of the formed spheroids was high and comparable to that observed in the above Test Examples.

In summary, it was found that the cell culture sheet of the present invention can be prepared simply, facilitates cell culture operation, and enables more homogeneous production of cellular tissue structures.

### INDUSTRIAL APPLICABILITY

The cell culture sheet of the present invention can be prepared simply and assists efficient cell culture operation, and therefore can preferably be used in the field of cell-based pharmaceutical preparations, for example, spheroid-containing pharmaceutical preparations etc.

### REFERENCE SIGNS LIST

- 1: Cell Culture Sheet
- 11: Recess
- 11a: Inner circumferential face of a recess
- 11b: Bottom face of a recess
- 12: Sheet surface
- 21: Non-cell adhesive substance
- 22: Cell-adhesive substance
- 23: Adhesive layer

## Claims

1. A cell culture sheet comprising a plurality of recesses each having an opening of 1000 µm or less in diameter,
wherein each recess has an inner circumferential face and a bottom face,
wherein the inner circumferential face has a non-cell adhesive surface, and
wherein the bottom face has a cell-adhesive surface.

2. The cell culture sheet according to claim 1, wherein the cell culture sheet is a laminate comprising a layer having the non-cell adhesive surface and a layer having the cell-adhesive surface, wherein the layer having the non-cell adhesive surface has through-holes.

3. The cell culture sheet according to claim 2, further comprising an adhesive layer between the layer having the non-cell adhesive surface and the layer having the cell-adhesive surface.

4. The cell culture sheet according to any one of claims 1 to 3, wherein the cell-adhesive surface comprises a synthetic resin.

5. The cell culture sheet according to any one of claims 1 to 4, wherein the cell-adhesive surface comprises a resin composition containing a polyimide.

6. The cell culture sheet according to any one of claims 1 to 5, wherein the recesses are formed in a shape tapered to the bottom face.

7. The cell culture sheet according to any one of claims 1 to 6, wherein the number of the recesses formed per unit area (cm²) is 10 to 1000.

8. A method for producing a cell culture sheet, the method comprising laminating of a layer having a non-cell adhesive surface and a layer having a cell-adhesive surface, wherein the layer having a non-cell adhesive surface has a plurality of through-holes of 1000 µm or less in diameter.

9. A cell culture tool, comprising the cell culture sheet according to any one of claims 1 to 7.

10. A method for culturing spheroids, the method comprising culturing spheroids using the cell culture sheet or cell culture tool according to any one of claims 1 to 7 and 9.
